# NEUE EUROPÄISCHE PATENTSCHRIFT

(11) **EP 2 649 137 B2**
(45) Veröffentlichungstag und Bekanntmachung des Hinweises auf die Entscheidung über den Einspruch: **25.11.2020**
(45) Hinweis auf die Patenterteilung: 03.01.2018
(21) Anmeldenummer: 11788139.1
(22) Anmeldetag: 24.11.2011
(51) Int. Cl.: C09D 5/08, C23F 11/14, A61K 8/44

(54) **N-ACYLAMINOSÄUREN ALS KORROSIONSSCHUTZ**
N-ACYL AMINO ACID AS CORROSION PROTECTION
N-ACYL AMINO ACIDES COMME PROTECTION CONTRE LA CORROSION

(30) Priorität: 10.12.2010 DE 102010062807
(43) Veröffentlichungstag der Anmeldung: 16.10.2013
(73) Patentinhaber: Henkel AG & Co. KGaA, 40589 Düsseldorf (DE)
(72) Erfinder: MÜLLER, Burkhard, 21075 Hamburg (DE); KAFTAN, Pamela, 22525 Hamburg (DE)
(86) Internationale Anmeldenummer: PCT/EP2011/070895
(87) Internationale Veröffentlichungsnummer: WO 2012/076340

(56) Entgegenhaltungen:
- EP-A1- 1 428 762
- EP-A1- 1 428 762
- EP-A2- 0 792 921
- WO-A1-01/27351
- WO-A1-99/59958
- WO-A1-2008/015076
- FR-A1- 2 877 842
- US-A- 4 600 530
- US-A- 5 030 385
- US-A- 5 032 318
- US-A- 6 010 708
- US-A1- 2004 248 994
- "ORAMIX L 30, Sodium lauryl sarcosinate", Seppic, July 2007 (2007-07), pages 1-15,
- Merkblatt INRS 'peintures en poudre', 2005

## Beschreibung

Die vorliegende Erfindung betrifft die Verwendung von N-Acylaminosäuren oder deren Salze zum Schutz von Metall vor Korrosion, sowie Produkte, die einen metallhaltigen Behälter umfassen, in welchem eine Zusammensetzung enthalten ist, umfassend mindestens eine N-Acylaminosäure (bzw. deren Salz) und Wasser.

Für diverse Produktarten, insbesondere für Produkte zur Anwendung im Haushalt und in der Kosmetik, stellt die Abgabeform als Spray oder Schaum eine wichtige und wesentliche Art der Applikationshilfe dar. Bekannt sind beispielsweise Reinigungsschäume, Reinigungssprays, Raumbeduftungssprays, Deosprays, Haarsprays oder Haarschäume. Sollen die Sprays oder Schäume aus einem unter Druck stehenden Aerosolbehälter abgegeben werden, sind die zu versprühenden bzw. zu verschäumenden Zusammensetzungen überwiegend in Aerosolbehältern aus Metall, wie beispielsweise aus Aluminium, konfektioniert.

Aerosolbehälter aus Metall lassen sich im Produktionsprozess gut befüllen und gasdicht verschließen. Direkt nach dem Produktionsprozess halten diese befüllten Aerosolbehälter dem beaufschlagten inneren Gasdruck in hervorragendem Maße stand. Erst nach einiger Zeit der Lagerung ergeben sich oft Dichtigkeitsprobleme, die meist durch Korrosion der Metallteile verursacht werden. Der permanente Kontakt einer beispielsweise zu versprühenden bzw. zu verschäumenden Zusammensetzung mit Metallbauteilen des Behälters begünstigt die Korrosion. Die Korrosion fördert z.B. Lochfraß an Wandungen eines Aerosolbehälters, was oft zu Undichtigkeiten des Aerosolbehälters führt. An den undichten Stellen treten Gas und Produkt aus, was die Funktionstüchtigkeit des Produkts sowie die Produktsicherheit senkt. Desweiteren kann Korrosion gleichfalls die Funktionstüchtigkeit des Ventils beeinträchtigen. Für den Bereich der Konsumentenprodukte vergehen von der Herstellung des Produkts bis zur Verwendung der letzten Produktreste mitunter mehrere Jahre. Daher muss für den Kunden unter anderem sichergestellt sein, dass das Produkt - umfassend Applikationssystem und die zu applizierende Zusammensetzung - keiner Veränderung durch Korrosion unterliegt und auch nach dieser Zeit noch einwandfrei sicher funktioniert.

Es hat in der Vergangenheit nicht an Lösungsversuchen für dieses Problem gefehlt. Eine Möglichkeit Metall vor Korrosion zu schützen bietet eine Innenbeschichtung der Metallwandung mit einem Lack. Im Sinne der Erfindung (vgl. DIN EN 927-1: 1996-10) ist Lack ein flüssiger oder pastenförmiger oder pulverförmiger (gegebenenfalls pigmentierter) Beschichtungsstoff, der, auf einen Untergrund aufgebracht, eine deckende Beschichtung mit schützenden, dekorativen oder sonstigen spezifischen technischen Eigenschaften ergibt. Meist werden zur Beschichtung des Metalls Flüssiglacke (i.e. Nasslacke) eingesetzt, wobei die Lacke selbst sowie die daraus resultierende Lackschicht organische Lösemittel enthalten. Organische Lösemittel sollen in Lacken aus ökologischen Gründen und aus Gründen der Arbeitssicherheit weitgehend vermieden werden. Unter einem Pulverlack versteht man im Sinne der Erfindung (vgl. DIN EN 971-1: 1996-09) pulverförmige, lösemittelfreie Beschichtungsstoffe, die nach dem Schmelzen und gegebenenfalls Einbrennen eine Beschichtung ergeben. Pulverlacke eignen sich ebenfalls zur Beschichtung von Aerosolbehältern. Allerdings weist die mittels Pulverlack erzielte Beschichtung durch Lufteinschlüsse eine vermehrte Porenbildung auf. Diese Poren bilden wiederum eine Kontaktmöglichkeit der zu versprühenden oder zu verschäumenden Zusammensetzung mit dem Metall und fördern die Korrosion.

Darüber hinaus entstehen zu einem gewissen Maße durch den zum Verschließen der Behälter üblichen Vercrimpvorgang Mikrorisse in der Innenbeschichtung Iackierter Aerosolbehälter. An diesen Mikrorissen der Innenbeschichtung bildet sich ebenso bevorzugt Korrosion aus.

Es wurde nun überraschend gefunden, dass durch Verwendung mindestens einer N-Acylaminosäure die Korrosion von Metallteilen (insbesondere von mit korrosiven Flüssigkeiten befüllten Vorrats- bzw. Abgabebehältnissen) erheblich reduziert bis verhindert wird.

Wässrige, N-Acylaminosäure-haltige Zusammensetzungen sind beispielsweise aus dem Bereich der Kosmetik im Rahmen der Haarpflege aus Hart, J. Roger; Levy, Edward F. Org. Chem. Div., W. R. Grace and Co., Nashua, NH, USA. Soap, Cosmetics, Chemical Specialties (1977), 53(8), 31-34 bekannt.

Aus der US 4,600,530 sind Korrosionsinhibitorzusammensetzungen, die zur Inhibierung der Korrosion in Aerosolprodukten geeignet sind und ein Gemisch aus einem Nitroalkan mit 1 bis 3 Kohlenstoffatomen und einem tertiären C12-14-Alkylammoniumcarboxylat umfassen, bekannt.

Ein erster Gegenstand der Erfindung ist die Verwendung mindestens einer N-Acylaminosäure oder deren Salze zum Schutz von mit einem Pulverlack beschichteten metallischen Teilen eines Behälters vor Korrosion.

Unter Korrosion wird im Sinne der Erfindung die Reaktion eines metallischen Werkstoffs mit seiner Umgebung verstanden, die eine messbare Veränderung des Werkstoffs bewirkt und zu einer Beeinträchtigung der Funktion eines metallischen Bauteils oder eines ganzen Systems führen kann (vgl. DIN 50900-1: 1982-04, -2: 1984-01 und -3: 1985-09).

Aminosäuren sind Carbonsäuren mit einer oder mehrerer Aminogruppen. Gemäß Molekülstruktur der erfindungsgemäßen N-Acylaminosäuren bindet an mindestens eine der vorhandenen Aminogruppen der Aminosäure ein Acyl-Rest.

Wenn im folgenden auf eine N-Acylaminosäure verwiesen wird, so wird erfindungsgemäß ebenso deren Salzform impliziert.

Es ist bevorzugt, wenn der Acylrest der N-Acylaminosäure ein gesättigter oder ungesättigter, linearer oder verzweigter (C₈-C₃₀)-Acylrest ist. Dabei ist es wiederum bevorzugt, wenn sich der (C₈-C₃₀)-Acylrest ableitet von Caprinsäure, Caprylsäure, Laurinsäure, Stearinsäure, Ölsäure, Palmitinsäure, Linolsäure, Linolensäure, Fettsäuregemischen des Kokosöls, Fettsäuregemischen des Palmöls, Fettsäuregemischen des Tallöls oder Fettsäuregemischen des Rapsöls.

Bei den oben genannten Fettsäuregemischen handelt es sich um den jeweiligen Fettsäureschnitt der Glyzeride des entsprechend genannten Öls.

Bei den erfindungsgemäß verwendbaren N-Acylaminosäuren handelt es sich bevorzugt um N-acylierte alpha-Aminosäuren. Diese N-acylierten Aminosäuren können unter N-acylierten aliphatischen Aminosäuren (insbesondere N-acyliertem Glycin, N-acyliertem Alanin, N-acyliertem Valin, N-acyliertem Leucin, N-acyliertem Isoleucin), N-acylierten aromatischen Aminosäuren (insbesondere N-acyliertem Phenylalanin, N-acyliertem Tyrosin, N-acyliertem Tryptophan), N-acylierten sauren Aminosäuren (insbesondere N-Acylglutaminsäure, N-Acylsarcosin oder N-Acylasparaginsäure) sowie N-acylierten basischen Aminosäuren (insbesondere N-acyliertem Arginin, N-acyliertem Lysin, N-acyliertem Histidin) ausgewählt werden Hierbei ist es wiederum bevorzugt, die N-Acylaminosäure aus mindestens einer sauren Aminosäure auszuwählen. Im Rahmen der vorgenannten Ausführungsformen sind jeweils wiederum die o.g. gesättigten oder ungesättigten, linearen oder verzweigten N-(C₈-C₃₀)-Acylreste bevorzugt, insbesondere die explizit genannten (*vide supra*)*.*

Besonders bevorzugt geeignete N-Acylaminosäuren werden aus mindestens einer Verbindung der Formel (I) ausgewählt 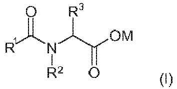 worin
- R¹: einen linearen oder verzweigten, gesättigten oder ungesättigten Kohlenwasserstoffrest bedeutet,
- R²: für ein Wasserstoffatom, eine (C₁ bis C₄)-Alkylgruppe oder eine (C₂ bis C₄)-Hydroxyalkylgruppe steht, 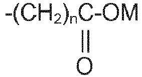
- R³: für ein Wasserstoffatom oder eine Gruppemit n = 1 oder 2,
- M: unabhängig voneinander ein Wasserstoffatom oder ein Äquivalent eines ein- oder mehrwertigen Kations bedeutet.

Beispiele für erfindungsgemäße (C₁ bis C₄)-Alkylgruppen sind Methyl, Ethyl, n-Propyl, Isopropyl, n-Butyl, sec-Butyl, iso-Butyl, tert-Butyl.
Beispiele für erfindungsgemäße (C₂ bis C₄)-Hydroxyalkylgruppen sind 2-Hydroxyethyl, 3-Hydroxypropyl, 2-Hydroxypropyl und 4-Hydroxybutyl.

Die Reste R¹ der Formel (I) stehen bevorzugt für eine (C₉ bis C₂₃)-Alkylgruppe, eine (C₉ bis C₂₃)-Alkenylgruppe mit bis zu vier ungesättigten Doppelbindungen oder eine (C₉ bis C₂₃)-Hydroxyalkylgruppe, besonders bevorzugt unabhängig voneinander für einen Rest, ausgewählt aus der Liste, die gebildet wird, aus Nonyl, Undecyl, Tridecyl, Pentadecyl, Heptadecyl, Nonadecyl, Henicosanyl, 15-Methylhexadecyl, Heptadec-8-enyl, Heptadeca-8,11-dienyl, Nonadeca-4,7,10,13-tetraenyl und Heptadeca-8,11,14-trienyl.

Der Rest R² gemäß Formel (I) steht bevorzugt für einen Rest, ausgewählt aus der Gruppe, die gebildet wird aus Wasserstoffatom, Methyl, Ethyl, Isopropyl, n-Propyl und 2-Hydroxyethyl. Besonders bevorzugt steht der besagte Rest R² für ein Wasserstoffatom oder eine Methylgruppe. Für den Fall, dass R³ für ein Wasserstoffatom steht, steht R² bevorzugt für eine Methylgruppe.Für den Fall, dass R³ für eine Gruppe 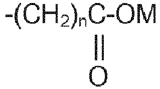 steht, steht R² bevorzugt für ein Wasserstoffatom.

Wenn die Verbindungen der Formel (I) als Säure vorliegen, bedeutet der Rest M ein Wasserstoffatom. Wenn die Verbindungen der Formel (I) als Salz vorliegen, stehen M für ein Äquivalent eines ein- oder mehrwertigen Kations. Das ein oder mehrwertige Kation M^{z+} mit jeweils einer Ladungszahl z von eins oder höher dient lediglich aus Gründen der Elektroneutralität zur Kompensation der einfach negativen Ladung des bei Salzbildung vorliegenden Carboxylatfragments - COO⁽⁻⁾ der Formel (I) . Das dafür zu verwendende Äquivalent des entsprechenden Kations beträgt 1/z. Das Fragment -COOM der Formel (I) steht im Fall der Salzbildung für die Gruppe:

-COO⁽⁻⁾ 1/z (M^{z+})

Als ein- oder mehrwertige Kationen M^{z+} kommen prinzipiell alle physiologisch verträglichen Kationen in Frage. Insbesondere sind dies Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (I) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M bzw. M' steht in den Formeln (I) bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Die Verbindungen der Formel (I) werden bevorzugt ausgewählt aus mindestens einer Verbindung aus der Gruppe, die gebildet wird, aus N-Lauroylsarcosin, N-Myristoylsarcosin, N-Palmitoylsarcosin, N-Oleylsarcosin, N-Cocoylsarcosin (wobei hier eine Mischung von Verbindungen vorliegt und Cocoyl der Zusammensetzung des Fettsäureschnitt des Kokosnussöls entspricht), N-Palmkernsarcosin (wobei hier eine Mischung von Verbindungen vorliegt und Palmkern der Zusammensetzung des Fettsäureschnitt des Palmkernöls entspricht), N-Lauroylglutamat, N-Myristoylglutamat, N-Palmitoylglutamat, N-Oleylglutamat, N-Cocoylglutamat (wobei hier eine Mischung von Verbindungen vorliegt und Cocoyl der Zusammensetzung des Fettsäureschnitt des Kokosnussöls entspricht), N-Palmkernglutamat (wobei hier eine Mischung von Verbindungen vorliegt und Palmkern der Zusammensetzung des Fettsäureschnitt des Palmkernöls entspricht), sowie aus den Salzen (insbesondere den Natriumsalzen) der vorgenannten Verbindungen.

Die Triglyceride des Kokosöls weisen folgende Fettsäurezusammensetzung auf:

| | |
|---|---|
| 45 bis 51 % | Laurinsäure |
| 16 bis 19 % | Tetradecansäure |
| 8 bis 10 % | Ölsäure |
| 9 bis 11 % | Palmitinsäure |
| 6 bis 9 % | Decansäure |
| 5 bis 8 % | Octansäure |

Die Triglyceride des Palmkernöls weisen folgende Fettsäurezusammensetzung auf:

| | |
|---|---|
| 47 bis 52 % | Laurinsäure |
| 16 bis 19 % | Tetradecansäure |
| 10 bis 18 % | Ölsäure |
| 6 bis 9 % | Palmitinsäure |
| 2 bis 3 % | Stearinsäure |
| 1 bis 3 % | Linolsäure |
| 2 bis 5 % | Decansäure |
| 1 bis 3 % | Octansäure |

Die besagte N-Acylaminosäure wird im Rahmen einer bevorzugten Ausführungsform zur Verringerung der Korrosion durch flüssige Zusammensetzungen verwendet. Zu diesem Zweck kann die N-Acylaminosäure als Beschichtung auf dem Metall aufgebracht, oder in die flüssige Zusammensetzung eingebracht werden. Im Rahmen einer besonders bevorzugten Ausführungsform der Erfindung liegt die N-Acylaminosäure bzw. deren Salz bevorzugt in einem zumindest bei einer Temperatur von 10 bis 40°C bei 1013 mbar flüssigen Medium gelöst vor. Bei dem flüssigen Medium handelt es sich bevorzugt um ein wasserhaltiges, flüssiges Medium, insbesondere um eine wasserhaltige Elektrolytlösung. Als Elektrolyt wird im Sinne der Erfindung eine chemische Verbindung verstanden, die in Lösung des entsprechenden wasserhaltigen, flüssigen Mediums in Ionen dissoziiert vorliegt. Besagte Elektrolyte sind von den besagten N-Acylaminosäuren verschieden.

Ein zweiter Erfindungsgegenstand ist ein Produkt, umfassend einen Behälter, der zumindest bereichsweise einen Hohlraum umschließt, wobei der Behälter zumindest ein metallisches Teil aufweist und wobei in dem Hohlraum eine Zusammensetzung, die mindestens eine N-Acylaminosäure, mindestens ein kationisches Tensid und Wasser umfasst, angeordnet ist und wobei metallische Teile des Behälters, die zum Hohlraum gewandt sind, mit einem Pulverlack beschichtet wurden.

Bevorzugt einsetzbare N-Acylaminosäuren sind die des ersten Erfindungsgegenstandes.

Die Zusammensetzung enthält die N-Acylaminosäure bevorzugt in einer Menge von 0,0001 bis 5,0 Gew.-%, besonders bevorzugt von 0,005 bis 2,0 Gew.-%, ganz besonders bevorzugt von 0,005 bis 1,0 Gew.-%, jeweils bezogen auf das Gewicht der Zusammensetzung.

Die Zusammensetzung enthält bevorzugt mindestens 20 Gew.-% Wasser, besonders bevorzugt mindestens 40 Gew.-% Wasser, jeweils bezogen auf das Gewicht der Zusammensetzung.

Die in dem Hohlraum des Behälters befindliche Zusammensetzung ist bevorzugt zumindest bei einer Temperatur von 10 bis 40°C bei 1013 mbar flüssig.

Die in dem Hohlraum des Behälters befindliche Zusammensetzung enthält bevorzugt neben der N-Acylaminosäure zusätzliche Elektrolyte. Dabei kann es sich um organische und/oder anorganische Salze handeln, wie beispielsweise Natriumchlorid, anionische Tenside oder um Polyelektrolyte, wie beispielsweise ionische filmbildende und/oder ionische festigende Polymere (insbesondere entsprechende kationische Polymere, anionische Polymere oder amphotere Polymere). Die zusätzlichen Elektrolyte sind selbstredend von den N-Acylaminosäuren verschieden.
Die erfindungsgemäß eingesetzten N-Acylaminosäuren verringern in besonderem Maße das Korrosionspotenzial von Zusammensetzungen, die Halogenid-haltige Elektrolyte umfassen, insbesondere ausgewählt unter Chlorid- und Bromid-haltigen Elektrolyten.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Der erfindungsgemäße Korrosionsschutz eignet sich besonders für solche Zusammensetzungen, die neben Wasser zusätzlich mindestens ein kationisches Polymer enthalten. Unter einem Polymer wird erfindungsgemäß eine Substanz mit einer mittleren Molmasse (Gewichtsmittel) von größer 10000 g/mol verstanden, welche aus mindestens einer sich wiederholenden Struktureinheit aufgebaut und durch natürliche oder synthetische Polyreaktion (i.e. Umsetzung eines Monomeren oder eines Gemisches verschiedener Monomere) zugänglich ist.

Kationische Polymere weisen im Sinne der Erfindung mindestens eine Struktureinheit auf, die mindestens ein permanent kationisiertes Stichstoffatom enthält. Unter permanent kationisierten Stickstoffatomen sind solche Stickstoffatome zu verstehen, die eine positive Ladung tragen und dadurch eine quartäre Ammoniumverbindung bilden. Quartäre Ammonium-Verbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen.

Im Sinne der Erfindung bevorzugte Zusammensetzungen enthalten die kationischen Polymere in einer Menge von 0,1 Gew.-% bis 20,0 Gew.-%, besonders bevorzugt von 0,2 Gew.-% bis 10,0 Gew.-%, jeweils bezogen auf das Gewicht des Mittels.

Die kationischen Polymere werden erfindungsgemäß bevorzugt aus kationischen, quaternisierten Cellulose-Derivaten ausgewählt.

Es erweisen sich generell solche kationischen, quaternisierten Cellulosen als im Sinne der Erfindung vorteilhaft, die in einer Seitenkette mehr als eine permanente kationische Ladung tragen. Darunter sind unter den kationischen Cellulosederivaten solche hervorzuheben, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat® H 100, Celquat® L 200 von der Firma National Starch vertrieben werden.

Weiterhin eignen sich solche kationischen Polymere bevorzugt, die mindestens eine Struktureinheit der Formel (1) und mindestens eine Struktureinheit der Formel (2) und gegebenenfalls mindestens eine Struktureinheit der Formel (3) umfassen 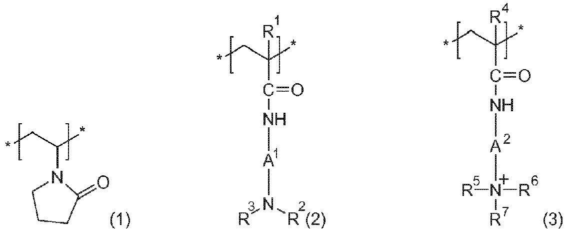 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

Zur Kompensation der positiven Ladung des Monomers (3) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Dabei kann es erfindungsgemäß bevorzugt sein, wenn die kationischen Polymere neben den Struktureinheiten der Formel (1) und der Formel (2) und der Formel (3) zusätzlich mindestens eine Struktureinheit der Formel (4) umfasst:

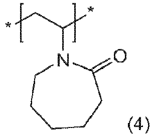

Geeignete Verbindungen sind beispielsweise als
- Copolymere aus mit Diethylsulfat quaterniertem Dimethylaminoethylmethacrylat, mit N-Vinylpyrrolidon mit der INCI-Bezeichnung Polyquaternium-11 unter den Bezeichnungen Gafquat® 440, Gafquat®734, Gafquat®755 (jeweils Firma ISP) sowie Luviquat PQ 11 PN (Firma BASF SE),
- Copolymere aus Methacryloylaminopropyllauryldimethylammonium chlorid mit N-Vinylpyrrolidon und Dimethylaminopropylmethacrylamid mit der INCI-Bezeichnung Polyquaternium-55 unter den Handelsnamen, Styleze W-10, Styleze® W-20 (Firma ISP),
- Copolymere aus Methacryloylaminopropyllauryldimethylammonium chlorid mit N-Vinylpyrrolidon, N-Vinylcaprolactam und Dimethylaminopropylmethacrylamid mit der INCI-Bezeichnung Polyquaternium-69 unter den Handelsnamen, Aquastyle® 300 (Firma ISP) im Handel erhältlich.

Zu den im Sinne der Erfindung bevorzugt geeignete kationische Polymere gehören ebenso solche kationischen Copolymere, die mindestens ein Strukturelement der Formel (M1) enthalten 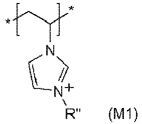 worin
R" für eine (C₁ bis C₄)-Alkylgruppe, insbesondere eine Methylgruppe, steht, und zusätzlich mindestens ein weiteres kationisches und/oder nichtionisches Strukturelement aufweisen.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist erfindungsgemäß bevorzugt, wenn in dem erfindungsgemäßen Mittel als kationisches Polymer mindestens ein Copolymer enthalten ist, das neben mindestens einem Strukturelement der Formel (M1) zusätzlich ein Strukturelement der Formel (1) umfasst
worin 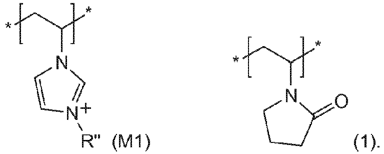 R" für eine (C₁ bis C₄)-Alkylgruppe, insbesondere eine Methylgruppe, steht.

Zur Kompensation der positiven Polymerladung der Copolymere dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Besonders Bevorzugte kationische Polymere enthalten 10 bis 30 Mol-%, vorzugsweise 15 bis 25 Mol.-% und insbesondere 20 Mol.-% Struktureinheiten gemäß Formel (M1) und 70 bis 90 Mol.-%, vorzugsweise 75 bis 85 Mol.-% und insbesondere 80 Mol.-% Struktureinheiten gemäß Formel (1).

Hierbei ist besonders bevorzugt, wenn die Copolymere neben Polymereinheiten, die aus dem Einbau der genannten Struktureinheiten gemäß Formel (M1) und (1) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere bis auf den Terminus ausschließlich aus Struktureinheiten der Formel (M1) mit R" = Methyl und (1) aufgebaut und lassen sich durch die allgemeine Formel (Poly1) 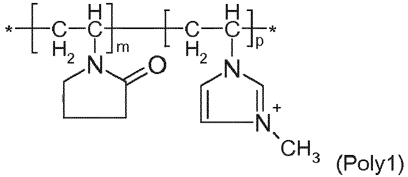 beschreiben, wobei die Indices m und p je nach Molmasse des Polymers variieren und nicht bedeuten sollen, daß es sich um Blockcopolymere handelt. Vielmehr können Struktureinheiten der Formel (M1) und der Formel (1) im Molekül statistisch verteilt vorliegen.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat® Style, Luviquat® FC 370, Luviquat® FC 550, Luviquat® FC 905 und Luviquat® HM 552.

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat® UltraCare erhältlich.

Besonders bevorzugte erfindungsgemäße Mittel enthalten ein Copolymer, insbesondere der Formel (Poly1), das Molmassen innerhalb eines bestimmten Bereiches aufweist. Hier sind erfindungsgemäße Mittel bevorzugt, bei denen das Copolymer, eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) oder an dessen bzw. deren Stelle können die erfindungsgemäßen Zusammensetzungen auch Copolymere enthalten, die neben Struktureinheiten der Formeln (M1-a) und (1) als zusätzliche Struktureinheiten diejenigen der Formel (4) aufweisen

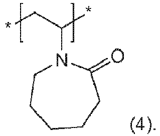

Weitere besonders bevorzugte erfindungsgemäße Mittel sind somit dadurch gekennzeichnet, daß sie als kationisches Polymer mindestens ein Copolymer enthalten, das mindestens eine Struktureinheit gemäß Formel (M1-a) und mindestens eine Struktureinheit gemäß Formel (1) und mindestens weitere Struktureinheit gemäß Formel (4) enthält

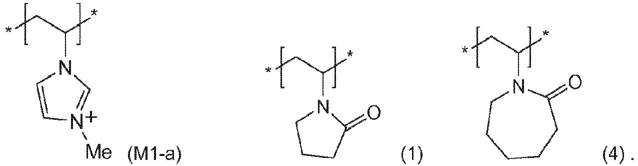

Auch hierbei ist besonders bevorzugt, wenn die Copolymere neben Polymereinheiten, die aus dem Einbau der genannten Struktureinheiten gemäß Formel (M1-a), (1) und (4) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere bis auf den Terminus ausschließlich aus Struktureinheiten der Formeln (M1-a), (1) und (4) aufgebaut und lassen sich durch die allgemeine Formel (Poly2) 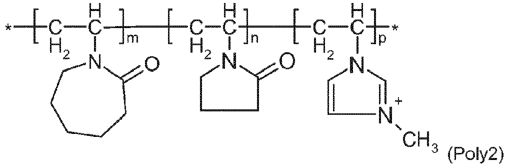 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen.

Zur Kompensation der positiven Polymerladung der Komponente dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymers der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat® Hold erhältlich.

Ganz besonders bevorzugte Copolymere enthalten 1 bis 20 Mol-%, vorzugsweise 5 bis 15 Mol.-% und insbesondere 10 Mol.-% Struktureinheiten gemäß Formel (M1-a) und 30 bis 50 Mol.-%, vorzugsweise 35 bis 45 Mol.-% und insbesondere 40 Mol.-% Struktureinheiten gemäß Formel (1) und 40 bis 60 Mol.-%, vorzugsweise 45 bis 55 Mol.-% und insbesondere 60 Mol.-% Struktureinheiten gemäß Formel (4).

Besonders bevorzugte erfindungsgemäße Mittel enthalten ein Copolymer, das Molmassen innerhalb eines bestimmten Bereiches aufweist. Hier sind erfindungsgemäße Mittel bevorzugt, bei denen das Copolymer eine Molmasse von 100 bis 1000 kDa, vorzugsweise von 250 bis 900 kDa, weiter bevorzugt von 500 bis 850 kDa und insbesondere von 650 bis 710 kDa aufweist.

Die erfindungsgemäßen Zusammensetzungen können als kationisches Polymer auch Copolymere enthalten, die als Struktureinheiten Struktureinheiten der Formeln (M1-a) und (1) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel sind dadurch gekennzeichnet, daß sie als kationisches Polymer mindestens ein Copolymer enthalten, das mindestens eine Struktureinheit gemäß Formel (M1-a) und mindestens eine Struktureinheit gemäß Formel (I) und mindestens eine Struktureinheit gemäß Formel (5) und mindestens eine Struktureinheit gemäß Formel (6) enthält

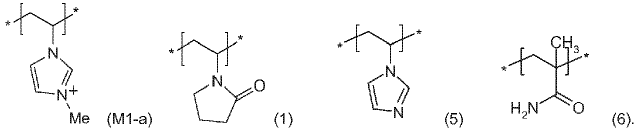

Auch hierbei ist besonders bevorzugt, wenn die Copolymere neben Polymereinheiten, die aus dem Einbau der genannten Struktureinheiten gemäß Formel (M1-a), (1), (5) und (6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere bis auf den Terminus ausschließlich aus Struktureinheiten der Formel (M1-a), (1), (5) und (6) aufgebaut und lassen sich durch die allgemeine Formel (Poly3) 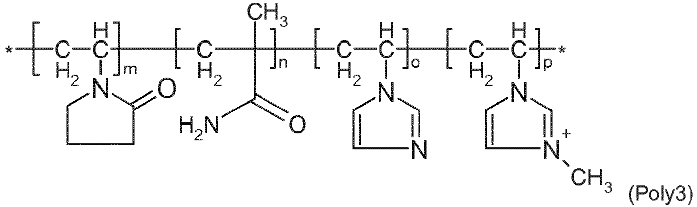 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M1-a), (1), (5) und (6) im Molekül statistisch verteilt vorliegen.

Zur Kompensation der positiven Polymerladung der Komponente dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymers der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat® Supreme erhältlich.

Ganz besonders bevorzugte Copolymere enthalten 1 bis 12 Mol-%, vorzugsweise 3 bis 9 Mol.-% und insbesondere 6 Mol.-% Struktureinheiten gemäß Formel (M1-a) und 45 bis 65 Mol.-%, vorzugsweise 50 bis 60 Mol.-% und insbesondere 55 Mol.-% Struktureinheiten gemäß Formel (1) und 1 bis 20 Mol.-%, vorzugsweise 5 bis 15 Mol.-% und insbesondere 10 Mol.-% Struktureinheiten gemäß Formel (5) und 20 bis 40 Mol.-%, vorzugsweise 25 bis 35 Mol.-% und insbesondere 29 Mol.-% Struktureinheiten gemäß Formel (6).

Besonders bevorzugte erfindungsgemäße Mittel enthalten ein Copolymer, das Molmassen innerhalb eines bestimmten Bereiches aufweist. Hier sind erfindungsgemäße Mittel bevorzugt, bei denen das Copolymer eine Molmasse von 100 bis 500 kDa, vorzugsweise von 150 bis 400 kDa, weiter bevorzugt von 250 bis 350 kDa und insbesondere von 290 bis 310 kDa aufweist.

Unter den zusätzlichen kationischen Polymeren mit mindestens einem Strukturelement der obigen Formel (M1), gelten als bevorzugt:
- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat® Style, Luviquat® FC 370, Luviquat® FC 550, Luviquat® FC 905 und Luviquat® HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat® Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat® Care oder Luviquat® Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat® Supreme (BASF SE)),
sowie Gemische aus diesen Polymeren.

Als kationische Tenside können erfindungsgemäß alle dem Fachmann bekannten und üblichen kationischen Tenside verwendet werden. Bevorzugt geeignete kationische Tenside sind permanent kationisch. Besonders bevorzugte kationische Tenisde werden ausgewählt unter:
- quartären Imidazolinverbindungen. Die im folgenden dargestellte Formel Quimi-I zeigt die Struktur dieser Verbindungen. 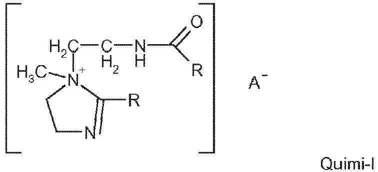 Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel I enthalten für R jeweils den gleichen Kohlenwasserstoffrest. Die Kettenlänge der Reste R beträgt bevorzugt 12 bis 21 Kohlenstoffatome. Besonders erfindungsgemäße Beispiele sind beispielsweise unter den INCI - Bezeichnungen Quaternium-27, Quaternium-72, Quaternium-83 und Quaternium-91 erhältlich.
- kationischen Tensiden gemäß der Formel (Tkat-2),

   RCO-X-N⁺R¹R²R³A⁻ (Tkat-2)

   R steht hierin für einen substituierten oder unsubstituierten, verzweigten oder geradkettigen Alkyl- oder Alkenylrest mit 11 bis 35 Kohlenstoffatomen in der Kette, X steht für - O - oder - NR⁵-,
   R¹ steht für eine Alkylengruppe mit 2 bis 6 C - Atomen, welche nicht substituiert oder substituiert sein kann, wobei im Falle einer Substitution die Substitution mit einer -OH - oder - NH- Gruppe bevorzugt ist, R², R³ jeweils unabhängig voneinander stehen für eine Alkyl oder Hydroxyalkylgruppe mit 1 bis zu 6 C - Atomen in der Kette, wobei die Kette geradlinig oder verzweigt sein kann.
   R⁵ steht für Wasserstoff oder einen C1 bis C6 geradkettigen oder verzweigten, Alkyl- oder Alkenylrest, welcher auch durch eine Hydroxygruppe substituiert sein kann.
   Innerhalb dieser Strukturklasse werden bevorzugt die Verbindungen einer der folgenden Strukturen verwendet:

   CH₃(CH₂)₂₀CONH(CH₂)₃ - N⁺(CH₃)₂-CH₂CH₃ A⁻ (Tkat-3)

   CH₃(CH₂)₂₀CONH(CH₂)₃ - N⁺(CH₃)₂-CH₂(CHOH)CH₂OHA- (Tkat-4)

   CH₃(CH₂)₂₀COOCH₂CHOHCH₂ - N⁺(CH₃)₃A⁻ (Tkat-5)

   CH₃(CH₂)₂₀CONH(CH₂)₃ - N⁺(CH₃)₂-CH₂CH₂OHA⁻ (Tkat-6)

   Beispiele für derartige Handelsprodukte sind Schercoquat BAS, Lexquat AMG-BEO, Akypoquat 131 oder Incroquat Behenyl HE.
- Esterquats gemäß der Formel (Tkat1-2) verwendet werden. 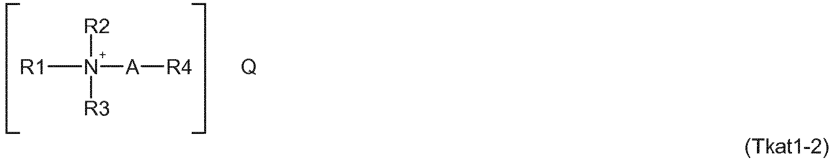 Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- A - R4), mit der Maßgabe, dass höchstens der Rest R1, R2 oder R3 für diesen Rest stehen können:
   Der Rest -(A - R4) ist mindestens 1 bis 3 mal enthalten.
      Hier steht A für:
      1) -(CH₂)ₙ- mit n = 1 bis 20, vorzugsweise n = 1 bis 10 und besonders bevorzugt n = 1 - 5, oder
      2) -(CHᵣCHR⁵-O)ₙ- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl, und
      R4 steht für:
      1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
      2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, und
      Q steht für ein physiologisch verträgliches organisches oder anorganisches Anion. Solche Produkte werden beispielsweise unter den Warenzeichen Rewoquat®, Stepantex®, Dehyquart® und Armocare® vertrieben. Die Produkte Armocare® VGH-70, ein N,N-Bis(2-Palmitoyloxyethyl)dimethylammonium-chlorid, sowie Dehyquart® F-75, Dehyquart® C-4046, Dehyquart® L80, Dehyquart® F-30, Dehyquart® AU-35, Rewoquat® WE18, Rewoquat® WE38 DPG und Stepantex® VS 90 sind Beispiele für solche Esterquats.
   Weitere erfindungsgemäß besonders bevorzugte Verbindungen der Formel (Tkat1-2) zählen zur Formel (Tkat1-2.1), den kationischen Betainestern. 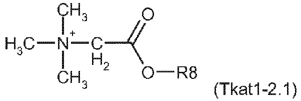 R8 entspricht in seiner Bedeutung R7.
- Monoalkyltrimethylammoniumsalzen mit einer Kettenlänge des Alkylrestes von 12 bis 24 Kohlenstoffatomen entsprechend der Formel (Tkat1-1), 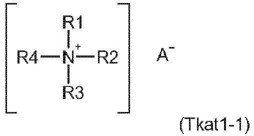 in denen R1, R2 und R3 für jeweils eine Methylgruppe stehen und R4 für einen gesättigten, verzweigten oder unverzweigten Alkylrest mit einer Kettenlänge von 12 bis 24 Kohlenstoffatomen steht und A- ein Anion bedeutet. Beispiele für Verbindungen der Formel (Tkat1-1) sind Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat.
- quaternisierten Amidoaminen mit der folgenden Strukturformel:

   R¹-NH-(CH₂)ₙ-N⁺R²R³R⁴ (Tkat7)

   worin R¹ ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und R2 , R3 und R4 jeweils unabhängig voneinander Wasserstoff oder ein Alkylrest mit 1 bis 4 C-Atomen, welcher gleich oder verschieden, gesättigt oder ungesättigt sein kann, und X- ein Anion und
   n eine ganze Zahl zwischen 1 und 10 bedeuten.
   Als erfindungsgemäß zu verwendende Amidoamine, welche quaternisiert sind, kommen beispielsweise in Betracht Rewoquat® RTM 50 (Witco Surfactants GmbH, INCI-Bezeichnung: Ricinoleamidopropyltrimonium Methosulfate), Empigen® CSC (Albright&Wilson, INCI-Bezeichnung: Cocamidopropyltrimonium Chlorid), Swanol® Lanoquat DES-50 (Nikko, INCI-Bezeichnung: Quatemium-33), Rewoquat® UTM 50 (Witco Surfactants GmbH, Undecyleneamidopropyltrimonium Methosulfate),
- kationischen Tensiden der Formel (Tkat-8) 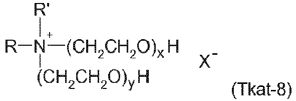 worin
   x und y stehen unabhängig voneinander für eine ganze Zahl größer 0,
   R steht für eine (C₈ bis C₂₀)-Alkylgruppe oder eine (C₈ bis C₂₀)-Alkenylgruppe,
   R' steht für eine Gruppe *-(CH₂CH₂O)_{z} H worin z eine ganze Zahl größer 0 bedeutet, eine (C₈ bis C₂₀)-Alkylgruppe oder eine (C₈ bis C₂₀)-Alkenylgruppe,
   X⁻ steht für ein Anion. Es ist erfindungsgemäß bevorzugt, wenn R' gemäß Formel (Tkat-8) für eine Gruppe *-(CH₂CH₂O)_{z}H steht, worin z eine ganze Zahl größer 0 bedeutet. Ein bevorzugtes kationisches Tensid der Formel (Tkat-8) ist das Tris(oligooxyethyl)alkylammonium-Dihydrogenphosphat-Salz mit dem Molekulargewicht von 780 g/mol, das mit der INCI-Bezeichnung Quaternium-52 beispielsweise unter dem Handelsnamen Dehyquart® SP von der Firma Cognis vertrieben wird.
Das Anion aller zuvor beschriebenen kationischen Tenside ist ausgewählt aus den physiologisch verträglichen Anionen. Beispielhaft hierfür seien beispielsweise die Halogenidionen, Fluorid, Chlorid, Bromid, Sulfat der allgemeinen Formel RSO₃⁻, worin R die Bedeutung von gesättigtem oder ungesättigtem Alkylresten mit 1 bis 4 Kohlenstoffatomen hat, oder anionische Reste organischer Säuren wie Maleat, Fumarat, Oxalat, Tartrat, Citrat, Lactat oder Acetat, genannt.

Es ist ganz besonders bevorzugt, wenn die erfindungsgemäße Zusammensetzung mindestens ein Monoalkyltrimethylammoniumsalz der Formel (Tkat1-1) enthält, 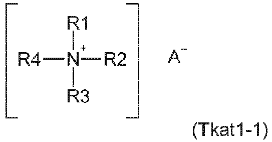 in denen R1, R2 und R3 für jeweils eine Methylgruppe stehen und R4 für einen gesättigten, verzweigten oder unverzweigten Alkylrest mit einer Kettenlänge von 12 bis 24 Kohlenstoffatomen steht und A⁻ ein Anion, insbesondere Chlorid oder Bromid, bedeutet.

Ganz besonders bevorzugte Verbindungen der Formel (Tkat1-1) werden ausgewählt unter Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid, Behenyltrimethylammoniummethosulfat.

Die zuvor genannten kationischen Tenside können einzeln oder in beliebigen Kombinationen miteinander verwendet werden.

Die Einsatzmengen der kationischen Tenside liegen bevorzugt zwischen 0,01 bis 20 Gew.%, besonders bevorzugt sind Mengen von 0,01 bis 10 Gew.% und ganz besonders bevorzugt sind Mengen von 0,1 bis 7,5 Gew.% enthalten. Die allerbesten Ergebnisse werden dabei mit Mengen von 0,1 bis 5 Gew. % erhalten. Alle Mengen beziehen sich jeweils auf das Gewicht der gesamten Zusammensetzung.

Die Zusammensetzungen können zusätzlich mindestens einen Monoalkohol mit 1 bis 4 Kohlenstoffatomen, wie zum Beispiel Ethanol, Isopropanol, enthalten.
Es ist somit möglich, mindestens einen (C₁ bis C₄)-Monoalkylalkohol in den erfindungsgemäßen Mitteln insbesondere in einer Menge von 1 bis 50 Gew.-% insbesondere von 5 bis 30 Gew.-% einzusetzen. Dies kann wiederum insbesondere für die Konfektionierung als Aerosolschaum bevorzugt sein.

Als zusätzliche Co-Solventien können mindestens ein organisches Lösungsmittel mit einem Siedepunkt unter 400°C oder mindestens ein Gemisch aus besagten Lösungsmitteln (wiederum bevorzugt in einer Menge von 0,1 bis 15 Gewichtsprozent, besonders bevorzugt von 1 bis 10 Gewichtsprozent bezogen auf die gesamte Zusammensetzung) enthalten sein.
Besonders bevorzugte wasserlösliche Lösungsmittel sind Glycerin, Ethylenglykol, Polyethylenglykol, Propylenglykol, Polypropylenglykol (wiederum bevorzugt in einer Menge bis 15 Gew.-% bezogen auf die gesamte Zusammensetzung).

Insbesondere der Zusatz von Glycerin und/oder Propylenglykol und/oder Polyethylenglykol und/oder Polypropylenglykol erhöht zusätzlich die Flexibilität des bei Anwendung der erfindungsgemäßen Zusammensetzung gebildeten Polymerfilms. Wird also ein besonders flexibler Halt gewünscht, enthalten die erfindungsgemäßen Zusammensetzungen vorzugsweise 0,01 bis 15 Gew.-% Glycerin und/oder Propylenglykol und/oder Polyethylenglykol und/oder Polypropylenglykol bezogen auf die gesamte Zusammensetzung.

Die Zusammensetzungen weisen bevorzugt einen pH-Wert von 2 bis 11 auf. Besonders bevorzugt liegt der pH-Bereich zwischen 2 und 7, ganz besonders bevorzugt zwischen 4 und 6. Die Angaben zum pH-Wert beziehen sich dabei im Sinne dieser Schrift auf den pH-Wert bei 25°C, sofern nichts anderes vermerkt ist.

Die erfindungsgemäßen Zusammensetzungen können weiterhin die Hilfs- und Zusatzstoffe enthalten, die üblicherweise herkömmlichen Stylingmitteln zugesetzt werden.
Als geeignete Hilfs- und Zusatzstoffe sind insbesondere zusätzliche Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Siliciumorganischer Verbindungen. Zunächst werden hierunter die Dimethiconole verstanden.
Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone bilden die zweite Gruppe der Silikone, welche erfindungsgemäß enthalten sein können. Diese können sowohl linear als auch verzweigt als auch cyclisch oder cyclisch und verzweigt sein.

Dimethiconcopolyole bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning® 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.
Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Besonders geeignete Silikone sind aminofunktionelle Silikone, insbesondere die Silikone, die unter der INCI-Bezeichnung Amodimethicone zusammengefasst sind. Daher ist es erfindungsgemäß bevorzugt, wenn die erfindungsgemäßen Mittel zusätzlich mindestens ein aminofunktionelles Silikon enthalten. Darunter sind Silikone zu verstehen, welche mindestens eine, gegebenenfalls substituierte, Aminogruppe aufweisen. Diese Silikone werden nach der INCI-Deklaration als Amodimethicone bezeichnet und sind beispielsweise in Form einer Emulsion als Handelsprodukt Dow Corning® 939 oder als Handelsprodukt Dow Corning® 949 im Gemisch mit einem kationischen und eine nichtionischen Tensid erhältlich.
Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Die Zusammensetzungen enthalten die Silikone bevorzugt in Mengen von 0,01 Gew.-% bis 15 Gew.-%, besonders bevorzugt von 0,05 bis 2 Gew.-%, bezogen auf die gesamte Zusammensetzung.

Als Pflegestoff einer anderen Verbindungsklasse kann das Mittel beispielsweise mindestens ein Proteinhydrolysat und/oder eines seiner Derivate enthalten.
Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.
Erfindungsgemäß können Proteinhydrolysate sowohl pflanzlichen als auch tierischen oder marinen oder synthetischen Ursprungs eingesetzt werden.
Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann die erfindungsgemäße Zusammensetzung weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.
Bevorzugt enthalten die erfindungsgemäßen Zusammensetzungen Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt.
Ganz besonders bevorzugt wird als Pflegestoff D-Panthenol, gegebenenfalls in Kombination mit mindestens einem der oben genannten Silikonderivate eingesetzt.

Der Zusatz von Panthenol erhöht die Flexibilität des bei Anwendung der Zusammensetzung gebildeten Polymerfilms. Wird also ein besonders flexibler Halt gewünscht, können die Zusammensetzungen Panthenol enthalten. In einer bevorzugten Ausführungsform enthalten die Zusammensetzungen Panthenol, vorzugsweise in einer Menge von 0,05 bis 10 Gew.-%, besonders bevorzugt 0,1-5 Gew.-%, jeweils bezogen auf die gesamte Zusammensetzung.

Als Pflegestoff können die Zusammensetzungen weiterhin mindestens einen Pflanzenextrakt enthalten.
Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Weiterhin kann es bevorzugt sein, in den erfindungsgemäßen Mitteln Mischungen aus mehreren, insbesondere aus zwei, verschiedenen Pflanzenextrakten einzusetzen.

Auch Mono- bzw. Oligosaccharide können als Pflegestoff in den erfindungsgemäßen Mitteln eingesetzt werden.
Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.
Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.
Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC® sowie Phospholipid SV® vertrieben. Die Zusammensetzungen enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.
Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol®OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 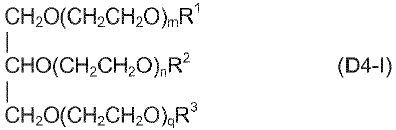 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl Die Zusammensetzungen selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt. Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält die erfindungsgemäße Zusammensetzung weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, kationische Azofarbstoffe, nichtionische Azofarbstoffe, Anthrachinone oder Indophenole. Die Zusammensetzungen gemäß dieser Ausführungsform enthalten die direktziehenden Farbstoffe bevorzugt in einer Menge von 0,001 bis 20 Gew.-%, bezogen auf die gesamte Zusammensetzung.

Es ist erfindungsgemäß bevorzugt, dass die Zusammensetzungen frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Zur Aufbewahrung in besagten Behältern eignen sich besonders bevorzugt Zusammensetzungen, die Wasser, mindestens eine N-Acylaminosäure und mindestens ein ionisches filmbildendes und/oder ionisches festigendes Polymer enthalten.

Zur Aufbewahrung in besagten Behältern eignen sich besonders bevorzugt die folgenden Zusammensetzungen (A) bis (K):

### (A):

Zusammensetzung, umfassend mindestens eine N-Acylaminosäure, mindestens ein kationisches Tensid, mindestens ein kationisches Polymer und Wasser.

### (B):

Zusammensetzung, umfassend mindestens eine N-Acylaminosäure ausgewählt aus mindestens einer Verbindung der Formel (I) 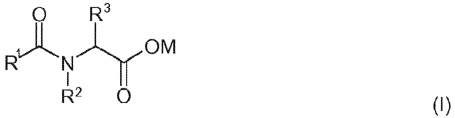 worin
- R¹: einen linearen oder verzweigten, gesättigten oder ungesättigten Kohlenwasserstoffrest bedeutet,
- R²: für ein Wasserstoffatom, eine (C₁ bis C₄)-Alkylgruppe oder eine (C₁ bis C₄)-Hydroxyalkylgruppe steht,
- R³: für ein Wasserstoffatom oder eine Gruppe 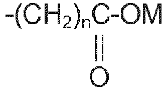 mit n = 1 oder 2,
- M: unabhängig voneinander ein Wasserstoffatom oder ein Äquivalent eines ein- oder mehrwertigen Kations bedeutet,
mindestens ein kationisches Tensid, mindestens ein kationisches Polymer und Wasser.

### (C):

Zusammensetzung, umfassend mindestens eine N-Acylaminosäure ausgewählt aus mindestens einer Verbindung der Formel (I) 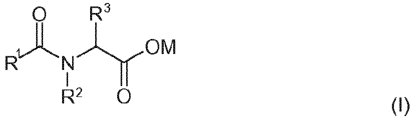 worin
- R¹: einen linearen oder verzweigten, gesättigten oder ungesättigten Kohlenwasserstoffrest bedeutet,
- R²: für ein Wasserstoffatom, eine (C₁ bis C₄)-Alkylgruppe oder eine (C₁ bis C₄)-Hydroxyalkylgruppe steht,
- R³: für ein Wasserstoffatom oder eine Gruppe 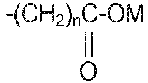 mit n = 1 oder 2,
- M: unabhängig voneinander ein Wasserstoffatom oder ein Äquivalent eines ein- oder mehrwertigen Kations bedeutet,
mindestens ein kationisches Tensid eines Monoalkyltrimethylammoniumsalzes der Formel (Tkat1-1), 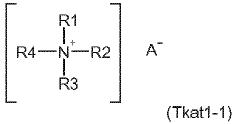 in denen R1, R2 und R3 für jeweils eine Methylgruppe stehen und R4 für einen gesättigten, verzweigten oder unverzweigten Alkylrest mit einer Kettenlänge von 16 bis 24 Kohlenstoffatomen und A- ein Anion bedeutet,
mindestens ein kationisches Polymer und Wasser.

### (D):

Zusammensetzung, umfassend Wasser, mindestens eine N-Acylaminosäure ausgewählt aus mindestens einer Verbindung der Formel (I) 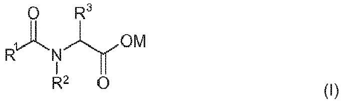 worin
- R¹: einen linearen oder verzweigten, gesättigten oder ungesättigten Kohlenwasserstoffrest bedeutet,
- R²: für ein Wasserstoffatom, eine (C₁ bis C₄)-Alkylgruppe oder eine (C₁ bis C₄)-Hydroxyalkylgruppe steht,
- R3: für ein Wasserstoffatom oder eine Gruppe 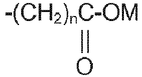 mit n = 1 oder 2,
- M: unabhängig voneinander ein Wasserstoffatom oder ein Äquivalent eines ein- oder mehrwertigen Kations bedeutet,
mindestens ein kationisches Tensid eines Monoalkyltrimethylammoniumsalzes der Formel (Tkat1-1), 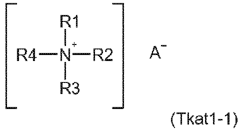 in denen R1, R2 und R3 für jeweils eine Methylgruppe stehen und R4 für einen gesättigten, verzweigten oder unverzweigten Alkylrest mit einer Kettenlänge von 16 bis 24 Kohlenstoffatomen und A- ein Anion bedeutet,
mindestens ein kationisches Polymer ausgewählt unter kationischen, quaternisierten Cellulose-Derivaten.

### (E):

Zusammensetzung, umfassend Wasser, mindestens eine N-Acylaminosäure ausgewählt aus mindestens einer Verbindung der Formel (I) 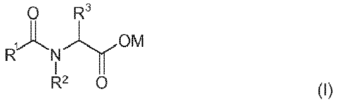 worin
- R¹: einen linearen oder verzweigten, gesättigten oder ungesättigten Kohlenwasserstoffrest bedeutet,
- R²: für ein Wasserstoffatom, eine (C1 bis C4)-Alkylgruppe oder eine (C1 bis C4)-Hydroxyalkylgruppe steht,
- R³: für ein Wasserstoffatom oder eine Gruppe 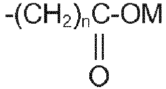 mit n = 1 oder 2,
- M: unabhängig voneinander ein Wasserstoffatom oder ein Äquivalent eines ein- oder mehrwertigen Kations bedeutet,
mindestens ein kationisches Tensid eines Monoalkyltrimethylammoniumsalzes der Formel (Tkat1-1), 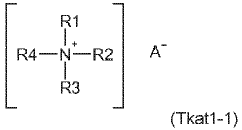 in denen R¹, R² und R³ für jeweils eine Methylgruppe stehen und R⁴ für einen gesättigten, verzweigten oder unverzweigten Alkylrest mit einer Kettenlänge von 16 bis 24 Kohlenstoffatomen und A⁻ ein Anion bedeutet,
mindestens ein kationisches Polymer, das mindestens eine Struktureinheit der Formel (1) und mindestens eine Struktureinheit der Formel (2) und gegebenenfalls mindestens eine Struktureinheit der Formel (3) umfasst 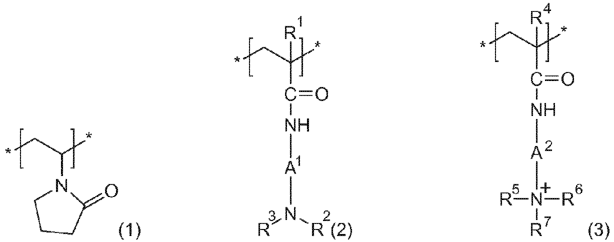 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

### (F):

Zusammensetzung, umfassend 0,0001 bis 5,0 Gew.-% mindestens einer N-Acylaminosäure, von 0,1 bis 7,5 Gew.% mindestens eines kationischen Tensids, von 0,1 Gew.-% bis 20,0 Gew.-% mindestens eines kationischen Polymers und Wasser.

### (G):

Zusammensetzung, umfassend Wasser, 0,0001 bis 5,0 Gew.-% mindestens einer N-Acylaminosäure ausgewählt aus mindestens einer Verbindung der Formel (I) 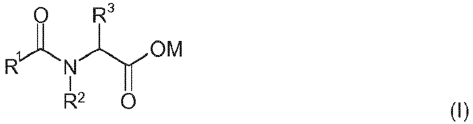 worin
- R¹: einen linearen oder verzweigten, gesättigten oder ungesättigten Kohlenwasserstoffrest bedeutet,
- R²: für ein Wasserstoffatom, eine (C1 bis C4)-Alkylgruppe oder eine (C1 bis C4)-Hydroxyalkylgruppe steht,
- R³: für ein Wasserstoffatom oder eine Gruppe 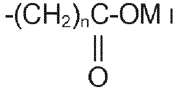 mit n = 1 oder 2,
- M: unabhängig voneinander ein Wasserstoffatom oder ein Äquivalent eines ein- oder mehrwertigen Kations bedeutet,
0,1 bis 7,5 Gew.% mindestens eines kationischen Tensids, und
0,1 Gew.-% bis 20,0 Gew.-% mindestens eines kationischen Polymers.

### (H):

Zusammensetzung, umfassend Wasser, 0,0001 bis 5,0 Gew.-% mindestens einer N-Acylaminosäure ausgewählt aus mindestens einer Verbindung der Formel (I) 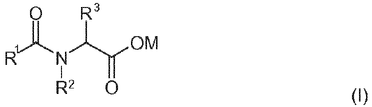 worin
- R¹: einen linearen oder verzweigten, gesättigten oder ungesättigten Kohlenwasserstoffrest bedeutet,
- R²: für ein Wasserstoffatom, eine (C₁ bis C₄)-Alkylgruppe oder eine (C₁ bis C₄)-Hydroxyalkylgruppe steht,
- R³: für ein Wasserstoffatom oder eine Gruppe 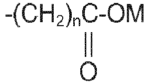 mit n = 1 oder 2,
- M: unabhängig voneinander ein Wasserstoffatom oder ein Äquivalent eines ein- oder mehrwertigen Kations bedeutet,
0,1 bis 7,5 Gew.% mindestens eines kationischen Tensids eines Monoalkyltrimethylammoniumsalzes der Formel (Tkat1-1), 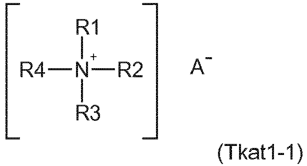 in denen R1, R2 und R3 für jeweils eine Methylgruppe stehen und R4 für einen gesättigten, verzweigten oder unverzweigten Alkylrest mit einer Kettenlänge von 12 bis 24 Kohlenstoffatomen und A⁻ ein Anion bedeutet, und
0,1 Gew.-% bis 20,0 Gew.-% mindestens eines kationischen Polymers.

### (J):

Zusammensetzung, umfassend Wasser, 0,0001 bis 5,0 Gew.-% mindestens einer N-Acylaminosäure ausgewählt aus mindestens einer Verbindung der Formel (I) 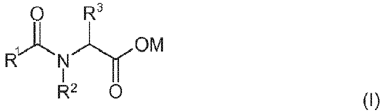 worin
- R¹: einen linearen oder verzweigten, gesättigten oder ungesättigten Kohlenwasserstoffrest bedeutet,
- R²: für ein Wasserstoffatom, eine (C₁ bis C₄)-Alkylgruppe oder eine (C₁ bis C₄)-Hydroxyalkylgruppe steht,
- R³: für ein Wasserstoffatom oder eine Gruppe 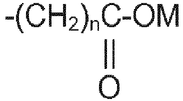 mit n = 1 oder 2,
- M: unabhängig voneinander ein Wasserstoffatom oder ein Äquivalent eines ein- oder mehrwertigen Kations bedeutet,
0,1 bis 7,5 Gew.% mindestens eines kationischen Tensids eines Monoalkyltrimethylammoniumsalzes der Formel (Tkat1-1), 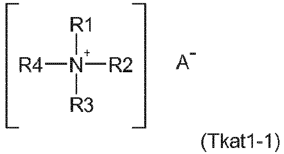 in denen R1, R2 und R3 für jeweils eine Methylgruppe stehen und R4 für einen gesättigten, verzweigten oder unverzweigten Alkylrest mit einer Kettenlänge von 12 bis 24 Kohlenstoffatomen und A⁻ ein Anion bedeutet, und
0,1 Gew.-% bis 20,0 Gew.-% mindestens eines kationischen Polymers ausgewählt unter kationischen, quaternisierten Cellulose-Derivaten.

### (K):

Zusammensetzung, umfassend Wasser, 0,0001 bis 5,0 Gew.-% mindestens einer N-Acylaminosäure ausgewählt aus mindestens einer Verbindung der Formel (I) 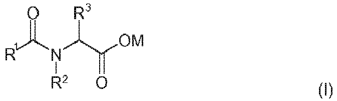 worin
- R¹: einen linearen oder verzweigten, gesättigten oder ungesättigten Kohlenwasserstoffrest bedeutet,
- R²: für ein Wasserstoffatom, eine (C1 bis C4)-Alkylgruppe oder eine (C1 bis C4)-Hydroxyalkylgruppe steht,

- R³: für ein Wasserstoffatom oder eine Gruppe 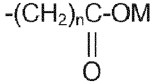 mit n = 1 oder 2,
- M: unabhängig voneinander ein Wasserstoffatom oder ein Äquivalent eines ein- oder mehrwertigen Kations bedeutet,
0,1 bis 7,5 Gew.% mindestens eines kationischen Tensids eines Monoalkyltrimethylammoniumsalzes der Formel (Tkat1-1), 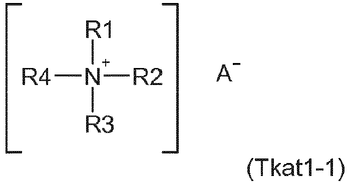 in denen R1, R2 und R3 für jeweils eine Methylgruppe stehen und R4 für einen gesättigten, verzweigten oder unverzweigten Alkylrest mit einer Kettenlänge von 12 bis 24 Kohlenstoffatomen und A⁻ ein Anion bedeutet, und
0,1 Gew.-% bis 20,0 Gew.-% mindestens eines kationischen Polymers, das mindestens eine Struktureinheit der Formel (1) und mindestens eine Struktureinheit der Formel (2) und gegebenenfalls mindestens eine Struktureinheit der Formel (3) umfasst 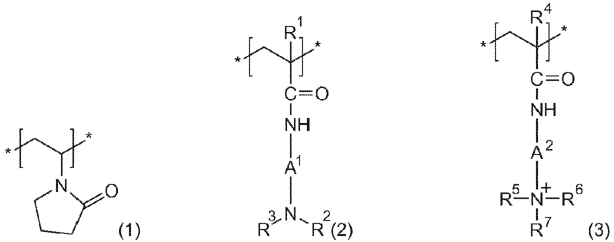 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

Die Zusammensetzungen (A) bis (K) enthalten bevorzugt mindestens 20 Gew.-% Wasser, besonders bevorzugt mindestens 40 Gew.-% Wasser, jeweils bezogen auf das Gewicht der Zusammensetzung.
Die Zusammensetzungen (A) bis (K) weisen bevorzugt einen pH-Wert von 2 bis 11, besonders bevorzugt zwischen 2 und 7, ganz besonders bevorzugt zwischen 4 und 6, auf.
Die Zusammensetzungen (A) bis (K) sind bevorzugt zumindest bei einer Temperatur von 10 bis 40°C bei 1013 mbar flüssig.
Die Zusammensetzungen (A) bis (K) enthalten bevorzugt zusätzlich mindestens ein Treibmittel.

Für die Zusammensetzungen (A) bis (K) gelten darüber hinaus für die darin genannten Merkmale alle zuvor genannten bevorzugten Parameter der Zusammensetzung (*vide supra*) mutatis mutandis als bevorzugt.

Es ist erfindungsgemäß bevorzugt, wenn der erfindungsgemäße Behälter vollständig einen Hohlraum umschließt, wobei sich in besagtem Hohlraum die besagte Zusammensetzung befindet.

Die Behälter umfassen als metallisches Teil bevorzugt mindestens eine zumindest teilweise den besagten Hohlraum umschliessende Wandung aus Metall. Als Metall eignet sich insbesondere Aluminium.

Die zum besagten Hohlraum gewandte Fläche (i.e. Innenseite) der Wandungen aus Metall ist mit einem Pulverlack beschichtet.

Zur Beschichtung wird ein Pulverlack verwendet. Pulverlacke werden im Allgemeinen in Pulverform oder als Schmelze auf die zu beschichtende Oberfläche des entsprechenden Metallteils des Behälters aufgebracht und dort gehärtet (thermisch, durch UV-Strahlung oder NIR-Strahlung). Als erfindungsgemäß verwendbare Pulverlacke eignen sich insbesondere Epoxidharze, Epoxidharz/Polyester-Gemische, Polyester, Polyester/Isocyanat-Gemische, Acrylate.

Pulverlacke basieren auf den Polymerklassen Epoxidharze, Epoxidharz/Polyester-Gemische, Polyester, Polyester/Isocyanat-Gemische und Acrylate. Die daraus gefertigten Pulverlacke heißen Epoxidharz-, Epoxidharz/Polyester-, Polyester-, Polyurethan- bzw. Acrylat-Pulverlacke.
Zur Namensgebung der Pulverlacktypen werden die zugrunde liegenden Polymerklassen ebenso herangezogen wie die Vernetzungschemie (Polyurethan-Pulverlack). Daraus resultieren manchmal irreführende Bezeichnungen. Es heißen z. B. Isocyanat-gehärtete Acrylate weiterhin Acrylate, während Isocyanat-gehärtete Polyester als Polyurethane bezeichnet werden.
Strahlenhärtende Pulverlacke runden die Palette der genannten, rein thermisch vernetzenden ab: Dabei sind die Nah-Infrarot härtenden NIR-Pulverlacke im Prinzip noch zu den thermisch härtenden zu zählen, denn sie können auch im Ofen gehärtet werden. Die UV-härtenden dagegen basieren auf einem rein strahlungshärtenden Bindemittel, benötigen also UV-Strahlung.
Füllstoffe beeinflussen den fertigen Lackfilm vor allem in seinen mechanischen Eigenschaften, also hinsichtlich Elastizität und Schlagtiefungsbeständigkeit sowie in der Chemikalienbeständigkeit. Aber auch Verlauf und Glanz und damit die dekorativen Aspekte lassen sich dadurch in breiten Grenzen steuern. Außerdem werden durch Füllstoffe unter anderem das Ausmaß der Kantendeckung und das Ablaufverhalten an den Kanten während des Beschichtens sowie die Dichte des Pulverlacks und damit seine Ergiebigkeit beeinflußt. Als Pulverlackfüllstoffe dienen vorwiegend natürliche Mineralien wie Schwerspate, Feldspäte und Kreiden.
Pulverlacke sind grundsätzlich in fast allen Farbtönen herstellbar. Für die Farbgebung sind dafür sowohl organische als auch anorganische Pigmente geeignet, sofern diese hinreichend temperaturstabil sind. Metall- und andere Effektpigmente können unter der hohen Scherbelastung bei der Herstellung von Pulverlacken leiden, so daß sie meist nach der Extrudierung in Pulverform eingearbeitet werden.

Eine weitere mögliche Ausführungsform der Erfindung ist dadurch gekennzeichnet, daß eine Pulverlackbeschichtung besagter Teile des Behälters mindestens einen Epoxidharz-Pulverlack umfaßt. Epoxidharz-Pulverlacke oder Epoxi-Pulverlacke sind Pulverlacke, in denen Epoxidharze oder epoxidierte Novolacke mit unterschiedlichen Härtern zu einer Beschichtung reagieren. Als Härter kommen je nach Anwendungsbereich Amine im weitesten Sinne oder modifiziertes Cyanoguanidin, Phenole wie Bisphenol A bzw. F oder ihre Derivate sowie Anhydrid-Härter bzw. zum Einsatz. Außer mit diesen niedermolekularen Härtern werden Epoxidharze auch mit sauren Polyesterharzen kombiniert, siehe weiter unten (Epoxidharz/Polyester-Pulverlacke). Beschichtungen, die aus Epoxidharz-Pulverlacken hergestellt werden, zeichnen sich durch gute mechanisch-technologische Werte aus.
Mit phenolischen Härtern werden extrem niedrige Einbrenntemperaturen erreicht. Amine, speziell die Dicyandiamid-Derivate, führen zu einer hohen Vernetzungsdichte des Pulverlackes. Für Anwendungen, in denen eine hohe Glasübergangstemperatur des vernetzten Films gefordert wird, eignen sich bevorzugt Anhydride. Diese sind in den meisten Fällen stark reizend und die daraus hergestellten Pulverzubereitungen kennzeichnungspflichtig. Ihr Einsatz erfolgt daher erfindungsgemäß weniger bevorzugt.

Eine weitere mögliche Ausführungsform der Erfindung ist dadurch gekennzeichnet, daß eine Pulverlackbeschichtung besagter Teile des Behälters mindestens einen Polyester-Pulverlack umfaßt.
Das klassische Polyester-System ist TGIC-haltig (Triglycidylisocyanurat). Polyester/TGIC-Pulverlacke werden bei Temperaturen oberhalb 150°C eingebrannt, meist bei ca. 190°C. Aufgrund ihrer geringen Vergilbungstendenz sind sogar Temperaturen bis 290°C möglich, so daß gegebenenfalls nur wenige Sekunden Objekttemperatur zur vollständigen Vernetzung des Lackfilms ausreichen.
Als weitere Vernetzungssysteme existieren zum einen die auf der Basis einer Polykondensation mit Hydroxylalkylamid vernetzenden Systeme, die sich durch geringe Einbrenntemperaturen und einen glatten Verlauf auszeichnen. Nachteilig ist jedoch die ausgeprägte Neigung zu Nadelstichen bei höheren Schichtdicken, die aus der Vernetzungsart (Abspaltung von Wasser) resultiert. Zum anderen sind seit einiger Zeit direkte Nachfolgetypen von TGIC im Einsatz. Wie TGIC-haltige Produkte vernetzen diese mittels einer Polyaddition mit dem Polyesterharz. So werden keine Abspaltprodukte freigesetzt, welche Nadelstiche verursachen können.
Eine bevorzugte erfindungsgemäße Ausführungsform ist dadurch gekennzeichnet, daß die besagte Pulverlackbeschichtung mindestens einen Hydroxylalkylamid vernetzenden Polyester-Pulverlack umfaßt.

Eine weitere bevorzugte Ausführungsform der erfindungsgemäßen Kombination ist dadurch gekennzeichnet, daß die Pulverlack-Innenbeschichtung mindestens einen Polyurethan-Pulverlack umfaßt.
PU-Pulverlacke sind Pulverlacke, in denen Hydroxy-Gruppen-tragende Polyester mit Isocyanaten gehärtet werden. Prinzipiell ist jedoch auch jedes andere Hydroxy-Gruppen-tragende Polymer als Bindemittel geeignet. Die Isocyanate sind meistens verkappt (blockiert), um einerseits einen leicht handhabbaren Feststoff einsetzen zu können, andererseits, um eine Vorreaktion von Bindemittel und Härter im Pulverlack zu vermeiden. Des Weiteren gewährleisten die Verkappungsmittel einen gefahrlosen Umgang mit dem Polyurethan-Pulverlack, da sie keinerlei freie Isocyanate enthalten. Die Verkappungsmittel - meistens Caprolactam - werden beim Einbrennen erst bei Temperaturen oberhalb 175°C abgespalten, so daß für die Vernetzung von Polyurethan-Pulverlack Objekttemperaturen von mindestens 180°C notwendig sind. Da die freigesetzten Verkappungsmittel ca. 5 % der eingesetzten Pulverlackmenge ausmachen, ist beim Einsatz solcher Pulverlacke auf eine effektive Abluftreinigung zu achten.

Polyurethan-Pulverlacke ergeben witterungs- und kreidungsbeständige Lackierungen, die bevorzugt für Außenanwendungen (Fassadenelemente, Automobilanbauteile) zum Einsatz kommen.

Eine weitere mögliche Ausführungsform der Erfindung ist dadurch gekennzeichnet, daß eine Pulverlackbeschichtung besagter Teile des Behälters mindestens einen Acrylat-Pulverlack umfaßt. Acrylat-Pulverlacke sind alle Pulverlacke mit einem Acrylatharz-Anteil im Bindemittel, wobei die Vernetzungschemie nicht in die Namensgebung eingeht. Prinzipiell sind auch bei den Acrylat-Pulverlacken alle Reaktionen denkbar, die zum Aufbau eines Netzwerkes befähigt sind, nämlich Epoxid/Hydroxy-, Epoxid/Carboxy- und Hydroxy/Isocyanat-Reaktionen.
Aufgrund der chemischen Umgebung in Acrylatharzen sind für den Epoxid/Carboxy-Mechanismus, anders als bei den Epoxidharz/Polyester-Pulverlacken, Einbrenntemperaturen von 130°C ausreichend. Zwecks besserer Pulverlagerstabilität und höherer Arbeitsgeschwindigkeit wird aber meist bei 160-190°C eingebrannt. Die Isocyanat-vernetzenden Acrylat-Pulverlacke benötigen wie die Polyurethan-Pulverlacke auf Polyester-Basis 180°C Objekttemperatur.
Die für Pulverlacke typische Orangenschalenstruktur ist auf ein Minimum reduziert. Acrylat-Pulverlacke kommen insbesondere für die Beschichtung von Aluminiumteilen oder als Deckschicht bei einer Mehrschichtlackierung in Frage.

Eine weitere mögliche Ausführungsform der Erfindung ist dadurch gekennzeichnet, daß eine Pulverlackbeschichtung besagter Teile des Behälters mindestens einen Hybridpulverlack aus miteinander vernetzenden Epoxid- und Polyesterharzen umfasst.
Bei Hybrid-Pulverlacken werden Epoxid- und Polyesterharze miteinander vernetzt.

Die vorliegende Erfindung bewährt sich insbesondere für die Bereitstellung von Aerosolprodukten umfassend mindestens einen Aerosolbehälter der mit mindestens einer metallischen Wandung einen Hohlraum umschließt, wobei in dem Hohlraum eine Zusammensetzung angeordnet ist, umfassend mindestens eine N-Acylaminosäure, mindestens ein Treibmittel, mindestens ein kationisches Tensid und Wasser. Insbesondere bevorzugt sind dabei die erfindungsgemäß in dem besagten Aerosolbehälter enthaltenen Zusammensetzungen als Aerosolschaum oder Aerosolspray (besonders bevorzugt als Aerosolschaum) konfektioniert.

Im Sinne der Erfindung wird unter einem Aerosolbehälter definitionsgemäß ein Behälter verstanden, dessen Innendruck höher ist als der Aussendruck seiner Umgebung. Die in einem Aerosolbehälter konfektionierten Mittel lassen sich beispielsweise versprühen oder verschäumen. Als "Nichtaerosolbehälter" wird im Umkehrschluß zur Aerosoldefinition ein Behältnis definiert, dessen Innendruck gleich dem Aussendruck seiner Umgebung ist. Die in einem Nichtaerosolbehälter konfektionierten Mittel lassen sich ausgießen oder mittels mechanischer Einwirkung durch ein Quetsch- oder Pumpsystem ausbringen.

Erfindungsgemäß geeignete Treibmittel sind beispielsweise ausgewählt aus N2 O, N2, Dimethylether, CO2 Luft, Alkanen mit 3 bis 5 Kohlenstoffatomen, wie Propan, n-Butan, iso-Butan, n-Pentan und isoPentan, und deren Mischungen. Bevorzugt sind Dimethylether, Propan, n-Butan, iso-Butan und Mischungen daraus.

Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.
Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 20 bis 98 Gew.-%, bezogen auf das gesamte Produkt, eingesetzt. Mengen von 30 bis 95 Gew.-%, insbesondere von 30 bis 65 Gew.-% sind besonders bevorzugt.

Die Aerosolprodukte lassen sich in üblicher Art und Weise herstellen. In der Regel werden alle Bestandteile der besagten Zusammensetzung mit Ausnahme des Treibmittels in einen geeigneten druckfesten Behälter eingefüllt.
Dieser wird daraufhin mit einem Ventil verschlossen. Über herkömmliche Techniken wird schließlich die gewünschte Menge Treibmittel eingefüllt.

Besonders bevorzugte Ausführungsformen der vorliegenden Erfindung sind die Aerosolprodukte (AA) und (BB):

### (AA):

Aerosolprodukt, umfassend einen Behälter, der mit mindestens einer, mit einem Pulverlack beschichteten, metallischen Wandung einen Hohlraum umschließt, wobei in dem Hohlraum eine Zusammensetzung angeordnet ist, die mindestens eine N-Acylaminosäure, mindestens ein Treibmittel, mindestens ein kationisches Tensid und Wasser umfasst.

### (BB):

Aerosolprodukt, umfassend einen Behälter, der mit mindestens einer, mit einem Pulverlack beschichteten, metallischen Wandung einen Hohlraum umschließt, wobei in dem Hohlraum eine Zusammensetzung angeordnet ist, die mindestens eine N-Acylaminosäure, mindestens ein Treibmittel, mindestens ein kationisches Tensid und Wasser umfasst.

Es ist wiederum besonders bevorzugt, wenn die Aerosolprodukte (AA) und (BB) als in dem Hohlraum befindliche Zusammensetzung, die als zuvor gekennzeichneten bevorzugten Zusammensetzungen umfassen. Insbesondere umfassen die Zusammensetzungen der Aerosolprodukte (AA) und (BB) bevorzugt neben dem Treibmittel die in den Zusammensetzungen (A) bis (K) definierten Parameter. Ferner eignen sich die Zusammensetzungen der Ausführungsform des dritten Erfindungsgegenstandes (*vide infra*) in bevorzugtem Maße.

Die erfindungsgemäßen Zusammensetzungen bzw. die Aerosolprodukte, die diese Mittel enthalten, insbesondere Aerosolhaarschäume bzw. Aerosolhaarsprays, zeichnen sich neben der hervorragenden Korrosionsstabilität des metallischen Behälters insbesondere dadurch aus, dass sie behandeltem Haar einen sehr starken, dauerhaften Frisurenhalt verleihen, obgleich das Haar flexibel bleibt. Wird das Mittel als Haarschaum konfektioniert, bildet sich ein stabiler, feinporiger und cremiger Schaum, der sich gleichmäßig und ohne zu tropfen auf dem Haar verteilen lässt.

Bevorzugte Zusammensetzungen, deren Korrosionswirkung durch den erfindungsgemäßen Gehalt an besagter N-Acylaminosäure eingedämmt bzw. verhindert wird, sind Zusammensetzungen zur temporären Verformung keratinischer Fasern, insbesondere menschlicher Haare, umfassend
(i) Wasser,
(ii) mindestens eine N-Acylaminosäure,
(iii) als Polyelektrolyt mindestens ein ionisches filmbildendes und/oder ionisches festigendes Polymer (insbesondere mindestens ein kationisches, filmbildendes und/oder kationisches festigendes Polymer),
(iv) mindestens ein Treibmittel,
(V) mindestens ein kationisches Tensid.

Unter keratinischen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Bevorzugte ionische filmbildende und/oder ionische festigende Polymere werden ausgewählt unter kationischen, anionischen oder amphoteren Polymeren sowie deren Gemischen. Kationische, filmbildende und/oder kationische festigende Polymere sind erfindungsgemäß besonders bevorzugt. Dabei werden die kationischen, filmbildenden und/oder kationischen, festigenden Polymere wiederum bevorzugt aus mindestens einem filmbildenden und/oder festigenden Polymer der kationischen Polymere des zweiten Erfindungsgegenstandes ausgewählt.

### Beispiele

Es wurden die Zusammensetzungen V1 (Vergleichszusammensetzung) und E1 (erfindungsgemäße Zusammensetzung) der Tabelle 1 nach Standardherstellverfahren bereitgestellt. Alle Mengenangaben sind - wenn nicht anders gekennzeichnet - Gewichtsprozent. Folgende Handelsprodukte wurden als Rohstoffe verwendet:

| | |
|---|---|
| Celquat® L 200 | quaterniertes Cellulose-Derivat (INCI-Bezeichnung: Polyquaternium-4) (National Starch) |
| Luviskol VA 64 W | Copolymer aus 60% N-Vinylpyrrolidon und 40% Vinylacetat mit einem K-Wert von 26-34, 50 Gew.-% Aktivsubstanz in Wasser) |
| Gafquat® 755 N | Dimethylaminoethylmethacrylat-Vinylpyrrolidon-Copolymer, quaterniert mit Diethylsulfat (ca. 19% Festkörper in Wasser; INCI-Bezeichnung: Polyquaternium-11) (ISP) |

**Tabelle 1: Zusammensetzungen**

| | V1 | E1 |
|---|---|---|
| Celquat L-2000 | 0,19 | 0,19 |
| Luviskol VA 64 W | 8,10 | 8,10 |
| Gafquat 755 N | 4,00 | 4,00 |
| 1,2-Propandiol | 3,40 | 3,40 |
| Glyzerin | 2,00 | 2,00 |
| PEG-8 (Polyethylenglykol, 400 g/mol) | 1,50 | 1,50 |
| Ethanol | 18,40 | 18,40 |
| D-Panthenol | 0,20 | 0,20 |
| Benzophenon-4 | 0,10 | 0,10 |
| Cetyltrimethylammoniumchlorid | 0,30 | 0,30 |
| Sodium Lauroyl Sarcosinate | - | 0,03 |
| PEG-40 Hydrogenated Castor Oil | 0,40 | 0,40 |
| Parfum | 0,10 | 0,10 |
| Milchsäure | ad pH 5 | ad pH 5 |
| Propan/Butan | 8,00 | 8,00 |
| Wasser | ad. 100 | ad. 100 |

Die treibmittelfreien Zusammensetzungen (d.h. ohne Propan/Butan-Anteil) wurden in Aerosoldosen aus Aluminium (Firma Aerocan) mit Pulverlack-beschichteten Innenwandungen (Pulverlack: Metlac 81200) gegeben und mit einem Tellerventil durch Vercrimpung verschlossen. Anschließend wurden die verschlossenen Behälter mit dem Treibmittel Propan/Butan beaufschlagt. Die erhaltenen Aerosolprodukte wurden über einen Zeitraum von 3 Monaten gelagert. Nach Ablauf der Lagerzeit wurden die Aerosoldosen entleert und zur Bewertung der Korrosionsphänomene aufgeschnitten. Die Pulverlackbeschichtung der erfindungsgemäßen Zusammensetzung E1 wies kaum durch Korrosion verursachte Blasen auf, während die Pulverlackbeschichtung des Aerosolprodukts der Vergleichzusammensetzung V1 erhebliche Korrosionserscheinungen und erhebliche Blasenbildung der Lackschicht zeigte.

## Patentansprüche

1. Verwendung mindestens einer N-Acylaminosäure oder deren Salze zum Schutz von mit einem Pulverlack beschichteten, metallischen Teilen eines Behälters vor Korrosion.

2. Verwendung nach Anspruch 1, **dadurch gekennzeichnet, daß** die N-Acylaminosäure aus mindestens einer Verbindung der Formel (I) ausgewählt wird 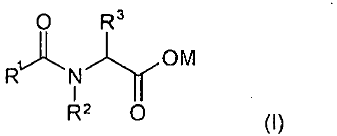 worin
R¹ einen linearen oder verzweigten, gesättigten oder ungesättigten Kohlenwasserstoffrest bedeutet,
R² für ein Wasserstoffatom, eine (C₁ bis C₄)-Alkylgruppe oder eine (C₂ bis C₄)-Hydroxyalkylgruppe steht,
R³ für ein Wasserstoffatom oder eine Gruppe 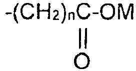 mit n = 1 oder 2,
M unabhängig voneinander ein Wasserstoffatom oder ein Äquivalent eines ein- oder mehrwertigen Kations bedeutet.

3. Produkt, umfassend einen Behälter, der zumindest bereichsweise einen Hohlraum umschließt, wobei der Behälter zumindest ein metallisches Teil aufweist und wobei in dem Hohlraum eine Zusammensetzung, die mindestens eine N-Acylaminosäure, mindestens ein kationisches Tensid und Wasser umfasst, angeordnet ist, **dadurch gekennzeichnet, dass** metallische Teile des Behälters, die zum Hohlraum gewandt sind, mit einem Pulverlack beschichtet wurden.

4. Produkt nach Anspruch 3, **dadurch gekennzeichnet, dass** die Zusammensetzung die N-Acylaminosäure bevorzugt in einer Menge von 0,0001 bis 5,0 Gew.-%, besonders bevorzugt von 0,005 bis 2,0 Gew.-%, ganz besonders bevorzugt von 0,005 bis 1,0 Gew.-%, jeweils bezogen auf das Gewicht der Zusammensetzung, enthält.

5. Produkt nach einem der Ansprüche 3 oder 4, **dadurch gekennzeichnet, dass** die Zusammensetzung bei einer Temperatur von 10 bis 40°C bei 1013 mbar flüssig ist.

6. Produkt nach einem der Ansprüche 3 bis 5, **dadurch gekennzeichnet, dass** die Zusammensetzung zusätzlich mindestens ein kationisches Polymer enthält.

7. Produkt nach einem der Ansprüche 3 bis 6, **dadurch gekennzeichnet, dass** der Behälter ein Aerosolbehälter ist und die Zusammensetzung zusätzlich mindestens ein Treibmittel enthält.

## Claims

1. Use of at least one N-acyl amino acid or the salts thereof in order to protect metal parts of a container, on which parts a powder coating is applied, from corrosion.

2. Use according to claim 1, **characterized in that** the N-acyl amino acid is selected from at least one compound of formula (I) 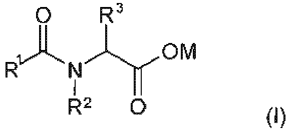 wherein
R¹ is a linear or branched, saturated or unsaturated hydrocarbon functional group,
R² is a hydrogen atom, a (C₁ to C₄) alkyl group or a (C₂ to C₄) hydroxyalkyl group,
R³ is a hydrogen atom or a 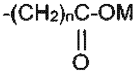 group where n = 1 or 2,
M is, independently of one another, a hydrogen atom or an equivalent of a monovalent or polyvalent cation.

3. Product, comprising a container that surrounds a cavity at least in regions, the container comprising least one metal part, and a composition which comprises at least one N-acyl amino acid, at least one cationic surfactant and water being arranged in the cavity, **characterized in that** a powder coating has been applied to metal parts of the container that face the cavity.

4. Product according to claim 3, **characterized in that** the composition contains the N-acyl amino acid preferably in an amount of from 0.0001 to 5.0 wt.%, particularly preferably from 0.005 to 2.0 wt.%, very particularly preferably from 0.005 to 1.0 wt.%, in each case based on the weight of the composition.

5. Product according to one of claims 3 or 4, **characterized in that** the composition is liquid at a temperature between 10 and 40°C at 1013 mbar.

6. Product according to one of claims 3 to 5, **characterized in that** the composition additionally contains at least one cationic polymer.

7. Product according to one of claims 3 to 6, **characterized in that** the container is an aerosol container and the composition additionally contains at least one propellant.

## Revendications

1. Utilisation d'au moins un N-acylaminoacide ou de ses sels pour protéger contre la corrosion des parties métalliques d'un contenant enduites d'une peinture en poudre.

2. Utilisation selon la revendication 1, **caractérisée en ce que** le N-acylaminoacide est choisi dans au moins un composé de formule (I) 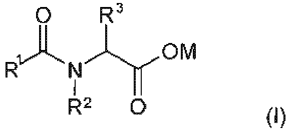 dans laquelle
R¹ représente un radical hydrocarboné linéaire ou ramifié, saturé ou insaturé,
R² est un atome d'hydrogène, un groupe alkyle en C₁ à C₄ ou un groupe hydroxyalkyle en C₂ à C₄,
R³ est un atome d'hydrogène ou un groupe 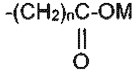 où n = 1 ou 2,
M représente, indépendamment, un atome d'hydrogène ou un équivalent d'un cation monovalent ou polyvalent.

3. Produit comprenant un contenant qui entoure au moins dans certaines zones une cavité, le contenant présentant au moins une partie métallique et une composition comprenant au moins un N-acylaminoacide, au moins un tensioactif cationique et de l'eau étant disposée dans la cavité, **caractérisé en ce que** des parties métalliques du contenant faisant face à la cavité ont été enduites d'une peinture en poudre.

4. Produit selon la revendication 3, **caractérisé en ce que** la composition contient le N-acylaminoacide de préférence en une quantité de 0,0001 à 5,0 % en poids, de manière particulièrement préférée de 0,005 à 2,0 % en poids, de manière tout particulièrement préférée de 0,005 à 1,0% en poids, respectivement par rapport au poids de la composition.

5. Produit selon l'une des revendications 3 ou 4, **caractérisé en ce que** la composition est liquide à une température de 10 à 40 °C à 1013 mbar.

6. Produit selon l'une des revendications 3 à 5, **caractérisé en ce que** la composition contient également au moins un polymère cationique.

7. Produit selon l'une des revendications 3 à 6, **caractérisé en ce que** le contenant est un contenant aérosol et **en ce que** la composition contient également au moins un agent gonflant.
